(19) Europäisches Patentamt
European Patent Office
Office européen des brevets

(11) Publication number: **0 026 030**
**B1**

(12) # EUROPEAN PATENT SPECIFICATION

(45) Date of publication of patent specification: **11.07.84**

(21) Application number: **80302431.4**

(22) Date of filing: **18.07.80**

(51) Int. Cl.[3]: **C 07 C 6/12**, C 07 C 2/86, C 07 C 15/08, C 07 C 15/04, B 01 J 29/28, C 01 B 33/28

(54) **Conversion of toluene over zeolite ZSM-48.**

(30) Priority: **03.08.79 US 63230**

(43) Date of publication of application:
**01.04.81 Bulletin 81/13**

(45) Publication of the grant of the patent:
**11.07.84 Bulletin 84/28**

(84) Designated Contracting States:
**BE DE FR GB IT NL**

(56) References cited:
**EP - A - 0 015 132**
**GB - A - 1 525 423**
**US - A - 3 965 210**
**US - A - 4 046 827**
**US - A - 4 160 788**

(73) Proprietor: **MOBIL OIL CORPORATION**
**150 East 42nd Street**
**New York New York 10017 (US)**

(72) Inventor: **Young, Lewis Brewster**
**Pineview Court**
**Skillman, New Jersey (US)**

(74) Representative: **West, Alan Harry et al,**
**Mobil Court 3 Clements Inn**
**London WC2A 2EB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 026 030 B1

**Description**

This invention relates to the use of a relatively new zeolite, ZSM—48, in catalytic hydrocarbon conversion processes, and more particularly to its use in selective production of *para*-disubstituted aromatics.

The disproportionation of aromatic hydrocarbons in the presence of zeolite catalysts has been described by Grandio *et al* in the *Oil and Gas Journal,* Vol. 69, No. 48 (1971).

U.S. Patents 3,126,422; 3,413,374; 3,598,878; 3,598,879 and 3,607,961 show vapor-phase disproportionation of toluene over various catalysts.

Alkylation of aromatic hydrocarbons with an olefin in the presence of a crystalline metallic aluminosilicate having uniform pore openings of about 6 to 15 Angstrom units (0.6 to 1.5 nm) is described in U.S. 2,290,607. U.S. 3,251,897 describes alkylation of aromatic hydrocarbons in the presence of X- or Y-type crystalline aluminosilicate zeolites, specifically such type zeolites wherein the cation is rare earth and/or hydrogen. U.S. 3,751,504 and 3,751,506 describe vapor phase alkylation of aromatic hydrocarbons with olefins, e.g. benzene with ethylene, in the presence of a ZSM—5 type zeolite catalyst.

In these prior art processes, the xylene product produced has the equilibrium composition of approximately 24 percent *para*, 54 percent of *meta* and 22 percent of *ortho*.

The alkylation of toluene with methanol in the presence of a cation exchanged zeolite Y has been described by Yashima *et al* in the Journal of Catalysis *16*, 273—280 (1970). These workers reported selective production of *p*-xylene over the approximate temperature range of 200 to 275°C. with the maximum yield of *p*-xylene in the mixture of xylenes, i.e. about 50 percent of the xylene product mixture, being observed at 225°C. Higher temperatures were reported to result in an increase in the yield of *m*-xylene and a decrease in the production of para- and ortho-xylene.

Transalkylation of toluene using a catalyst of faujasite or mordenite, a Group VIII metal, such as platinum, and an additional component of arsenic, antimony, bismuth, selenium, tellurium or compounds thereof is described in U.S. 3,527,824.

Of the xylene isomers, i.e. ortho, meta and para-xylene, meta xylene is the least desired product, with ortho and para-xylene being the more desired products. p-xylene is of particular value being useful in the manufacture of terephthalic acid which is an intermediate in the manufacture of synthetic fibers, such as "Dacron". Mixtures of xylene isomers, either alone or in further admixture with ethylbenzene, generally containing a concentration of about 24 weight percent para-xylene in the equilibrium mixture, have previously been separated by expensive superfractionation and multistage refrigeration steps. Such process, as will be realized, has involved high operation costs and has a limited yield.

The present invention is based on the observation that *para*-disubstituted aromatics such as *p*-xylene, *p*-ethyltoluene, and *p*-cymene are produced by alkylation catalyzed by a crystalline zeolite designated as ZSM—48. ZSM—48 is defined below and in our pending European application 80.300463 (published EP—Al—0015132).

According to the present invention, there is provided a process for converting toluene into products comprising a mixture of xylenes rich in *p*-xylene, which comprises contacting toluene, optionally together with an alkylating agent, at a temperature of 150 to 750°C, a pressure of 10.1325 to 10132.5 kPa and a weight hourly space velocity of 0.1 to 2000 with a catalyst comprising a catalytically-active form of the crystalline zeolitic material designated ZSM—48 having a lattice comprising $SiO_4$ tetrahedra crosslinked by the sharing of oxygen atoms having the compositional formula expressed in terms of moles of oxides per 100 moles of silica:

$$(0 \text{ to } 15)(RN)_2O: (0 \text{ to } 15)\ M_{2/n}O: (0 \text{ to } 2)\ Al_2O_3: (100)\ SiO_2$$

in which M is at least one cation of valence n and RN is a $C_1$ to $C_{20}$ organic compound having at least one amine functional group of pKa $\geqslant 7$, and being characterized by at least the following interplanar spacings and relative intensities of its X-ray diffraction pattern:

| d(A) | Relative Intensity |
|---|---|
| 11.8 ± 0.2 | W—VS |
| 10.2 ± 0.2 | W—M |
| 7.2 ± 0.15 | W |
| 4.2 ± 0.08 | VS |
| 3.9 ± 0.08 | VS |
| 3.6 ± 0.06 | W |
| 3.1 ± 0.05 | W |
| 2.85 ± 0.05 | W |

The X-ray data for ZSM—48 were determined by standard techniques. The radiation was the K-alpha doublet of copper, and a scintillation counter spectrometer with a strip chart pen recorder was used. The peak heights, I, and the positions as a function of 2 times theta, where theta is the Bragg angle, were read from the spectrometer chart. From these, the relative intensities, 100 $I/I_o$, where $I_o$ is the intensity of the strongest line or peak, and d(obs), the interplanar spacing in Angstrom units (1 Angstrom = 0.1 nm), corresponding to the recorded lines, were calculated. In Table 1, the relative intensities are given in terms of the symbols W = weak, VS = very strong and W—S = weak to strong. Ion exchange of the sodium ion with cations reveals substantially the same pattern with some minor shifts in interplanar spacing and variation in relative intensity. Other minor variations can occur depending on the silicon to aluminum ratio of the particular sample, as well as if it has been subjected to thermal treatment.

With regard to ZSM—48 zeolite, the original cations can be replaced, at least in part, by calcination and/or exchange with another cation. Thus, the original cations may be exchanged into a hydrogen or hydrogen ion precursor form or a form in which the original cation has been replaced by a metal of Groups 2 through 8 of the Periodic Table. Thus, for example, it is contemplated to exchange the original cations with ammonium ions or with hydronium ions. Catalytically active forms of these would include, in particular, hydrogen, rare earth metals, aluminum, metals of Groups II and VII of the Periodic Table and manganese.

An overview of the typical results obtained using ZSM—48 as a catalyst for several reactions is shown below:

| | | Wt % in Effluent | Isomer Dist. p | m | o |
|---|---|---|---|---|---|
| 2 | 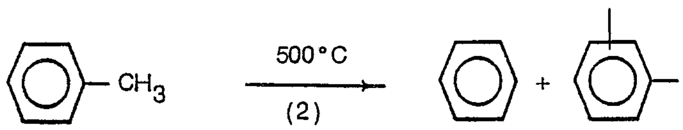 | 0.5<br>(Equil. | 33<br>24 | 51<br>54 | 16<br>22) |
| 4 | 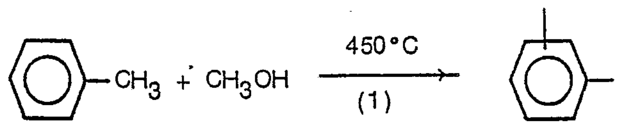 | 12.6<br>(Equil. | 50<br>24 | 30<br>54 | 20<br>22) |
| 5.2 | 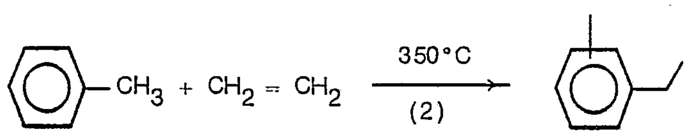 | 12.5<br>(Equil. | 47<br>32 | 52<br>50 | 1<br>18) |
| 5.3 | 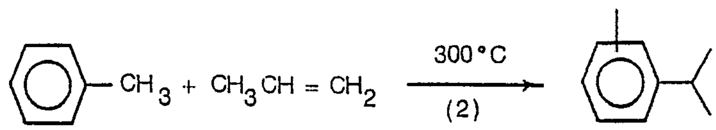 | 5.2<br>(Equil. | 67<br>29 | 28<br>69 | 5<br>2) |
| *m* + *p* | 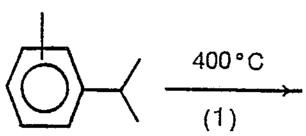 | *Para* 85% Conversion<br>*Meta* 7% Conversion | | | |

As can be seen, all reactions examined exhibited significant *para* selectivity.

Typical of the processes contemplated herein is the disproportionation of toluene to benzene and xylenes, wherein the proportion of para-xylene obtained is in excess of its normal equilibrium concentration. Such process is effectively carried out at a temperature of between about 350 and about 750°C at a pressure of between about 1 atmosphere (100 kPa) and about 100 atmospheres (10,000 kPa) utilizing a weight hourly space velocity of toluene of between about 0.25 and about 20.

Another process involves the methylation of toluene by reaction of the latter with a methylating agent, preferably methanol, at a temperature between about 250°C and about 750°C and preferably between about 325°C and about 600°C. The reaction generally takes place at atmospheric pressure, but the pressure may be within the approximate range of 1 atmosphere to 100 atmospheres (100 to 10,000 kPa). The molar ratio of methylating agent to toluene is generally between about 0.05 and about 5. When methanol is employed as the methylating agent a suitable molar ratio of methanol to toluene has been found to be approximately 0.1—2 moles of methanol per mole of toluene. With the use of other methylating agents, such as methylchloride, methylbromide, dimethylether, methylcarbonate, light olefins, or dimethylsulfide, the molar ratio of methylating agent to toluene may vary within the aforenoted range. Reaction is suitably accomplished utilizing a weight hourly space velocity of between about 0.1 and about 2000 and preferably between about 1 and about 100. The reaction product consisting predominantly of para-xylene or a mixture of para- and orthoxylene, together with comparatively smaller amount of meta-xylene may be separated by any suitable means, such as by passing the same through a water condenser and subsequently passing the organic phase through a column in which chromatographic separation of the xylene isomers is accomplished.

Other processes are:

Toluene + ethylene → p-ethyltoluene

Reagents and conditions are as follows:

Reagents: preferably ethylene; also ethanol, ethylether, ethylchloride, ethylbromide, etc.

Temperature: 200—600°C; preferably 300—500°C
Pressure: 0.1 atm—100 atm (100—10,000 kPa), preferably 1 atm—50 atm (100—5,000 kPa)
Molar ratio of ethylating agent to toluene of about 0.05—5; preferably 0.1—2
Weight hourly space velocity of toluene of about 1—100.

Toluene + propylene → p-cymene

Similarly, reagents and conditions are as follows:

Reagents: preferably propylene; also isopropylalcohol, isopropylether, isopropylchloride isopropyl bromide, etc.

Temperature: 150—500°C, preferably 200—400°C
Pressure: 0.1—100 atm (10—10,000 kPa); preferably 1—50 atm (100—5,000 kPa)
Molar ratios: propylene, etc./toluene 0.5—5; preferably 0.1—2
Weight hourly space velocity of toluene of about 1—100.

A still further charge stock which can be used in the process of the present invention to obtain high yields of para-xylene includes naphthenes, such as cyclopentane, cyclohexane and alkyl cyclopentanes having at least one alkyl group of 1 to 5 carbon atoms. Typical of the naphthene reactants are methyl cyclopentane, 1,2-dimethylcyclopentane, 1,3-dimethylcyclohexane. Another charge stock which can be effectively used in the present invention to selectively produce para-xylene includes paraffinic hydrocarbons having between 3 and 10 carbon atoms. Representative of such paraffins are butanes, pentanes, hexanes, heptanes, octanes and alkyl-substituted derivatives of these paraffins. Utilizing a paraffinic and/or naphthenic charge stock, reaction conditions include contact with the catalyst at a temperature of between about 400 to about 700°C, a pressure between about atmospheric (100 kPa) and about 1000 psig (7,000 kPa) and a weight hourly space velocity of 0.1 to 100.

The catalyst of this invention may be the product formed by impregnation of the zeolite powder or pellets with one or more alkaline earth compounds and/or ion exchanged with the same. Binders such as clays silica, or other inorganic oxides may be used. When such as used, the total catalyst composition should preferably contain at least 50 percent by weight of crystalline aluminosilicate zeolite. When the catalyst composition has the desired physical form, it is dried and then calcined at a temperature of at least about 1200°F (650°C) or higher, preferably in an oxidizing atmosphere such as air.

It is desirable to activate the catalyst by base exchange with ammonium salts followed by calcination in air at about 1000°F (538°C) for from about 15 minutes to about 24 hours.

The conversion processes described herein may be carried out as a batch type, semi-continuous or continuous operation utilizing a fixed or moving bed catalyst system. The catalyst after use may be conducted to a regeneration zone wherein coke is burned from the catalyst in an oxygen-containing atmosphere, e.g. air, at an elevated temperature, after which the regenerated catalyst is recycled to the conversion zone for further contact with the charge stock.

The following examples will serve to illustrate the process of the invention without limiting the same:

Examples 1 and 2

*Preparation of Catalyst* — A starting gel reaction mixture was prepared from sodium silicate (27.8% $SiO_2$, 8.4% $Na_2O$, 64% $H_2O$), $C_7$ (Example 1) and $C_8$ (Example 2) diamine compounds, sodium hydroxide and water. Crystallization was carried out in a stainless steel autoclave (160°C). After crystallization, the solids were separated from any unreacted components by filtration and then water washed followed by drying at about 100°C. Preparation of these samples is further described in EP—A1—0 015 132 and corresponds to Examples 6 and 11 of that application.

The product of Example 1 had a $SiO_2/Al_2O_3$ ratio of 1340 and a crystal size of about 1.0 micron, while for the product of Example 2, $SiO_2/Al_2O_3$ was 400 and crystal size was about 0.25 micron.

As synthesized materials were worked up by calcining in helium atmosphere at 500°C for 4 hours, ammonium exchanging twice in 2*N* $NH_4NO_3$, and calcining in air at 500°C before use.

Catalyst testing was done on 4.0 grams of catalyst at atmospheric pressure in quartz microreactors.

Examples 3—8

The following reactions were conducted using the catalysts produced by the methods of Examples 1 and 2 which serve as further examples of the invention.

Example 3 — Selective Toluene Disproportionation
Example 4 — Alkylation of Toluene with Methanol
Example 5 — Alkylation of Toluene with Ethylene
Example 6 — Alkylation of Toluene with Ethylene
Example 7 — Alkylation of Toluene with Propylene
Example 8 — Alkylation of Toluene with Propylene

Results obtained from the exercise of the foregoing examples are listed below in Tables 2—7, respectively.

TABLE II
TOLUENE DISPROPORTIONATION

Catalyst: Product of Example 2 (ZSM—48)
$SiO_2/Al_2O_3 = 400$
Feed: Toluene

| Run | 1 | 2 | 3 |
|---|---|---|---|
| Temp. °C | 400 | 500 | 550 |
| WHSV | 5.8 | 5.8 | 5.8 |
| Product, % | | | |
| Lt Gas | .005 | .020 | .029 |
| Benzene | .052 | .384 | 1.016 |
| Toluene | 99.844 | 99.080 | 97.699 |
| *para*-Xylene | .035 | .157 | .369 |
| *meta*-Xylene | .026 | .245 | .643 |
| *ortho*-Xylene | .010 | .081 | .230 |
| $C_9$+ | .029 | .033 | .014 |
| p/m/o XYL | 49/37/14 | 32.5/50.7/16.8 | 29.7/51.8/18.5 |

Pressure: 1 atmosphere (100 kPa)

TABLE III

ALKYLATION OF TOLUENE WITH METHANOL

Catalyst: Product of Example 1 (ZSM—48)
$SiO_2/Al_2O_3 = 1340$
Feed: Toluene/Methanol = 4 (molar ratio)

| Run | 1 | 2 | 3 | 4[a] | 5 |
|---|---|---|---|---|---|
| Temp. °C | 350 | 400 | 450 | 450 | 400 |
| WHSV | 5.8 | 5.8 | 5.8 | 18.6 | 6.2 |
| Product, % | | | | | |
| Lt Gas | 0.73 | 0.58 | 0.43 | 0.65 | 0.47 |
| Toluene | 95.44 | 89.72 | 85.07 | 94.96 | 88.24 |
| Ethylbenzene | 0.03 | 0.03 | 0.03 | 0.03 | 0.03 |
| *para*-Xylene | 1.87 | 4.38 | 6.33 | 2.20 | 5.20 |
| *meta*-Xylene | 0.93 | 2.44 | 3.71 | 1.09 | 2.81 |
| *ortho*-Xylene | 0.73 | 1.80 | 2.52 | 0.80 | 1.94 |
| $C_9$+ | 0.26 | 1.06 | 1.90 | 0.27 | 1.31 |
| Xylenes p | 53.0 | 50.8 | 50.4 | 53.8 | 52.3 |
| m | 26.4 | 28.3 | 29.5 | 26.6 | 28.2 |
| o | 20.7 | 20.9 | 20.1 | 19.6 | 19.5 |

[a] Methanol runs to 500°C + 3.5 hr calcination at 550°C prior to this run.

TABLE IV

ALKYLATION OF TOLUENE WITH ETHYLENE

Catalyst: Product of Example 1 (ZSM—48)
$SiO_2/Al_2O_3$ = 1340
Feed: Toluene/Ethylene = 5.3 (molar ratio)

| Run | 1 | 2 | 3 |
|---|---|---|---|
| Temp. °C | 350 | 450 | 550 |
| Tol WHSV | 6.0 | 6.0 | 6.0 |
| Tol/Ethylene (Mole) | 5.3 | 5.3 | 5.3 |
| Product, % | | | |
| Lt Gas | 0.32 | 0.29 | 0.27 |
| Benzene | 0.02 | 0.03 | 0.11 |
| Toluene | 98.55 | 98.43 | 98.72 |
| Ethylbenzene | — | 0.01 | 0.01 |
| *para*-Xylene | 0.05 | 0.03 | 0.06 |
| *meta*-Xylene | 0.02 | 0.02 | 0.05 |
| *ortho*-Xylene | 0.01 | 0.01 | 0.01 |
| *para*-Ethyltoluene | 0.70 | 0.80 | 0.38 |
| *meta*-Ethyltoluene | 0.16 | 0.37 | 0.38 |
| *ortho*-Ethyltoluene | — | — | 0.01 |
| n-Pro-Tol | 0.17 | 0.02 | — |
| p/m/o — ET | 81/19/0 | 68/32/0 | 50/49/1 |
| Tol Conv., % | 1.1 | 1.3 | 1.0 |

TABLE V

ALKYLATION OF TOLUENE WITH ETHYLENE

Catalyst: Product of Example 2 (ZSM—48)
$SiO_2/Al_2O_3 = 400$
Feed: Toluene/Ethylene = 5.2 (molar ratio)

| Run | 1 | 2 | 3 |
|---|---|---|---|
| Temp °C | 350 | 450 | 400 |
| WHSV Tol | 6.0 | 6.0 | 6.0 |
| Tol/Ethylene (Mole) | 5.2 | 5.2 | 5.2 |
| Product, % | | | |
| Lt Gas | 0.41 | 0.39 | 0.41 |
| Benzene | 0.04 | 0.16 | 0.06 |
| Toluene | 86.37 | 84.93 | 84.04 |
| Ethylbenzene | 0.08 | 0.10 | 0.06 |
| *para*-Xylene | 0.07 | 0.09 | 0.05 |
| *meta*-Xylene | 0.04 | 0.12 | 0.04 |
| *ortho*-Xylene | 0.02 | 0.04 | — |
| *para*-Ethyltoluene | 5.85 | 4.64 | 6.04 |
| *meta*-Ethyltoluene | 6.43 | 8.49 | 8.80 |
| *ortho*-Ethyltoluene | 0.19 | 0.94 | 0.43 |
| n-Pro-Tol | 0.47 | 0.12 | 0.06 |
| p/m/o — ET | 46.9/51.6/1.5 | 33.0/60.3/6.7 | 39.6/57.6/2.8 |
| Tol Conv., % | 13.3 | 14.7 | 15.6 |

TABLE VI

ALKYLATION OF TOLUENE WITH PROPYLENE

Catalyst: Product of Example 1 (ZSM—48)
$SiO_2/Al_2O_3 = 1340$
Feed: Toluene/Propylene

| Run | | 1 | 2 | 3 | 4 |
|---|---|---|---|---|---|
| Temp °C | | 240 | 300 | 350 | 345 |
| WHSV, Tol | | 5.4 | 5.4 | 5.4 | 1.7 |
| Tol/propylene, Mole | | 4.8 | 4.8 | 4.8 | 5 |
| Liq. Prod., % | | | | | |
| Lt Ends | | 3.30 | 4.30 | 4.38 | 3.72 |
| Toluene | | 95.78 | 93.90 | 93.53 | 92.81 |
| | | — | 0.06 | 0.06 | 0.08 |
| Cymenes | o | 0.083 | 0.046 | — | 0.074 |
| | m | 0.162 | 0.139 | 0.115 | 0.306 |
| | p | 0.672 | 1.437 | 1.502 | 2.104 |
| n-Pro-Toluene | | — | — | 0.424 | 0.908 |
| p/m/o-Cymenes | | 73/18/9 | 89/9/3 | 93/7/— | 85/12/3 |
| Tol Conv., % | | 1.0 | 1.9 | 2.2 | 3.6 |

TABLE VII

ALKYLATION OF TOLUENE WITH PROPYLENE

Catalyst: Product of Example 2 (ZSM—48)
$SiO_2/Al_2O_3 = 400$
Feed: Toluene/Propylene 5.3 (molar ratio)

| Run | | 1 | 2 |
|---|---|---|---|
| Temp. °C | | 300 | 350 |
| WHSV, Tol | | 5.9 | 5.9 |
| Tol/propylene (Mole) | | 5.3 | 5.3 |
| Product, % | | | |
| Lt Ends | | 3.46 | 4.18 |
| Toluene | | 87.65 | 88.29 |
| EB, Xyl | | .17 | .41 |
| Cymenes | o | .317 | .308 |
| | m | 1.436 | 1.234 |
| | p | 3.475 | 1.401 |
| n-Pro-Tol, etc. | | 3.50 | 4.18 |
| Cymenes | p | 66.5 | 47.6 |
| | m | 27.5 | 41.9 |
| | o | 6.1 | 10.5 |

The examples show selectivity for formation of *para*-isomers substantially in excess of equilibrium.

**Claims**

1. A process or converting toluene into products comprising a mixture of xylenes rich in p-xylene, which comprises contacting toluene, optionally together with an alkylating agent, at a temperature of 150 to 750°C, a pressure of 10.1325 to 10132.5 kPa and a weight hourly space velocity of 0.1 to 2000 with a catalyst comprising a catalytically-active form of the crystalline zeolitic material designated ZSM—48 having a lattice comprising $SiO_4$ tetrahedra crosslinked by the sharing of oxygen atoms having the compositional formula expressed in terms of moles of oxides per 100 moles of silica:

$$(0 \text{ to } 15)(RN)_2O: (0 \text{ to } 15)\ M_{2/n}O: (0 \text{ to } 2)\ Al_2O_3: (100)\ SiO_2$$

in which M is at least one cation of valence n and RN is a $C_1$ to $C_{20}$ organic compound having at least one amine functional group of $pKa \geqslant 7$, and being characterized by at least the following interplanar spacings and relative intensities of its X-ray diffraction pattern:

| d(A) | Relative Intensity |
|---|---|
| 11.8 ± 0.2 | W—VS |
| 10.2 ± 0.2 | W—M |
| 7.2 ± 0.15 | W |
| 4.2 ± 0.08 | VS |
| 3.9 ± 0.08 | VS |
| 3.6 ± 0.06 | W |
| 3.1 ± 0.05 | W |
| 2.85 ± 0.05 | W |

2. A process according to claim 1 wherein toluene is disproportionated at a temperature of 350 to 750°C, a pressure of 10.325 to 10132.5 kPa and a weight hourly space velocity of 0.25 to 20.

3. A process according to claim 1 wherein toluene and a methylating agent are contacted with the catalyst at a temperature of 250 to 750°C, a pressure of 10.1325 to 10132.5 kPa and a weight hourly space velocity of 0.1 to 2000, the molar ratio of methylating agent to toluene being 0.05 to 5.

4. A process according to claim 3 wherein the methylating agent is methanol and the contacting is effected at 325 to 600°C, 1 to 100 weight hourly space velocity and a methanol/toluene mol ratio of 0.1 to 2.

5. A process according to claim 1 wherein toluene and an ethylating agent are contacted with the catalyst at a temperature of 200 to 600°C, a pressure of 10.1325 to 10132.5 kPa, a weight hourly space velocity of 1 to 100 and an ethylating agent/toluene mole ratio of 0.5 to 5.

6. A process according to claim 5 wherein the ethylating agent is ethylene and the contacting is effected at 300 to 500°C, 10.1325 to 5066.25 kPa and an ethylene/toluene mol ratio of 0.1 to 2.

7. A process according to claim 1 wherein toluene and a propylating agent are contacted with the catalyst at a temperature of 150 to 500°C, a pressure of 10.1325 to 10132.5 kPa, a weight hourly space velocity of 1 to 100 and a propylating agent/toluene mol ratio of 0.5 to 5.

8. A process according to claim 7 wherein the propylating agent is propylene and the contacting is effected at 200 to 400°C, 10.1325 to 5066.25 kPa, and a propylene/toluene mol ratio of 0.1 to 2.

9. A process according to claim 3 wherein the methylating agent is methyl chloride or bromide, dimethyl ether, methyl carbonate, dimethylsulphide or light olefins.

10. A process according to claim 5 wherein the ethylating agent is ethanol, ethylether, or ethyl chloride or bromide.

11. A process according to claim 7 wherein the propylating agent is isopropyl alcohol, isopropyl ether or isopropyl chloride or bromide.

12. A process according to any preceding claim wherein the zeolite ZSM—48 has a silica/alumina ratio greater than 30.

13. A process according to any preceding claim wherein the zeolite ZSM—48 has a silica/alumina ratio greater than 50.

14. A process according to any preceding claim wherein the zeolite ZSM—48 has a silica/alumina ratio greater than 400.

15. A process according to any preceding claim wherein the zeolite ZSM—48 is in the hydrogen form.

**Patentansprüche**

1. Verfahren zur Umwandlung von Toluol in Produkte, die eine Mischung an Xylolen, die reich an p-Xylol ist, enthalten, das das Inkontaktbringen von Toluol, gegebenenfalls zusammen mit einem Alkylierungsmittel, bei einer Temperatur von 150 bis 750°C, einem Druck von 10,1325 bis 10132,5 kPa und einer stündlichen Gewichts-Raumgeschwindigkeit (whsv) von 0,1 bis 2000 mit einem Katalysator umfaßt, der eine katalytisch aktive Form des als ZSM—48 bezeichneten kristallinen zeolithischen Materials umfaßt, das ein Gitter aufweist, das $SiO_4$-Tetraeder umfaßt, die durch gemeinsame Sauerstoffatome miteinander vernetzt sind, und das, ausgedrückt in Molen Oxide pro 100 Mole Siliciumdioxid, die folgende Zusammensetzungs-Formel aufweist:

$$(0 \text{ bis } 15)\ (RN)_2O\text{: } (0 \text{ bis } 15)\ M_{2/n}O\text{: } (0 \text{ bis } 2)\ Al_2O_3\text{: } (100)\ SiO_2$$

in der M wenigstens ein Kation einer Wertigkeit n ist und RN eine organische Verbindung mit von 1 bis

20 Kohlenstoffatomen ist, die wenigstens eine funktionelle Amin-Gruppe mit einem pKa ≥ 7 aufweist, und das durch wenigstens die folgenden Netzebenenabstände und Relativ-Intensitäten seines Röntgenbeugungs-Bildes charakterisiert ist:

| d(A) | Relativintensität |
|---|---|
| 11,8 ± 0,2 | W—VS |
| 10,2 ± 0,2 | W—M |
| 7,2 ± 0,15 | W |
| 4,2 ± 0,08 | VS |
| 3,9 ± 0,08 | VS |
| 3,6 ± 0,06 | W |
| 3,1 ± 0,05 | W |
| 2,85 ± 0,05 | W |

2. Verfahren nach Anspruch 1, bei dem Toluol bei einer Temperatur von 350 bis 750°C, einem Druck von 10,325 bis 10132,5 kPa und einer stündlichen Gewichts-Raumgeschwindigkeit von 0,25 bis 20 disproportioniert wird.

3. Verfahren nach Anspruch 1, bei dem Toluol und ein Methylierungsmittel mit dem Katalysator bei einer Temperatur von 250 bis 750°C, einem Druck von 10,1325 bis 10132,5 kPa und einer stündlichen Gewichts-Raumgeschwindigkeit von 0,1 bis 2000 in Kontakt gebracht werden, wobei das molare Verhältnis des Methylierungsmittel zu dem Toluol 0,05 bis 5 beträgt.

4. Verfahren nach Anspruch 3, bei dem das Methylierungsmittel Methanol ist und das Inkontaktbringen bei 325 bis 600°C, einer stündlichen Gewichts-Raumgeschwindigkeit von 1 bis 100 und einem Methanol/Toluol-Molverhältnis von 0,1 bis 2 erfolgt.

5. Verfahren nach Anspruch 1, bei dem Toluol und ein Ethylierungsmittel mit dem Katalysator bei einer Temperatur von 200 bis 600°C, einem Druck von 10,1325 bis 10132,5 kPa, einer stündlichen Gewichts-Raumgeschwindigkeit von 1 bis 100 und einem Ethylierungsmittel/Toluol-Molverhältnis von 0,5 bis 5 in Kontakt gebracht werden.

6. Verfahren nach Anspruch 5, bei dem das Ethylierungsmittel Ethylen ist und das Inkontaktbringen bei 300 bis 500°C, 10,1325 bis 5066,25 kPa und einem Ethylen/Toluol-Molverhältnis bis 0,1 bis 2 erfolgt.

7. Verfahren nach Anspruch 1, bei dem Toluol und ein Propylierungsmittel mit dem Katalysator bei einer Temperatur von 150 bis 500°C, einem Druck von 10,1325 bis 10132,5 kPa, einer stündlichen Gewichts-Raumgeschwindigkeit von 1 bis 100 und einem Propylierungsmittel/Toluol-Molverhältnis von 0,5 bis 5 in Kontakt gebracht werden

8. Verfahren nach Anspruch 7, bei dem das Propylierungsmittel Propylen ist und das Inkontaktbringen bei 200 bis 400°C, 10,1325 bis 5066,25 kPa und einem Propylen/Toluol-Molverhältnis von 0,1 bis 2 erfolgt.

9. Verfahren nach Anspruch 3, bei dem das Methylierungsmittel Methylchlorid oder -bromid, Dimethylether, Methylcarbonat, Dimethylsulfid oder leichte Olefine sind.

10. Verfahren nach Anspruch 5, bei dem das Ethylierungsmittel Ethanol, Ethylether oder Ethylchlorid oder -bromid ist.

11. Verfahren nach Anspruch 7, bei dem das Propylierungsmittel Isopropylalkohol, Isopropylether oder Isopropylchlorid oder -bromid ist.

12. Verfahren nach einem beliebigen vorangehenden Anspruch, bei dem der Zeolith ZSM—48 ein Siliciumdioxid/Aluminiumoxid-Verhältnis von größer als 30 aufweist.

13. Verfahren nach einem beliebigen vorangehenden Anspruch, bei dem der Zeolith ZSM—48 ein Siliciumdioxid/Aluminiumoxid-Verhältnis von größer als 50 aufweist.

14. Verfahren nach einem beliebigen vorangehenden Anspruch, bei dem der Zeolith ZSM—48 ein Siliciumdioxid/Aluminiumoxid-Verhältnis von größer als 400 aufweist.

15. Verfahren nach einem beliebigen vorangehenden Anspruch, bei dem der Zeolith ZSM—48 in der Wasserstofform vorliegt.

**Revendications**

1. Un procédé pour la conversion du toluène en produits consistant en un mélange de xylènes riche en p-xylène, qui consiste à mettre en contact le toluène, facultativement avec un agent alkylant, à

une température de 150 à 750°C, sous une pression de 10,1325 à 10132,5 kPa et à une vitesse spatiale horaire en poids de 0,1 à 2000, avec un catalyseur consistant en une forme catalytiquement active de la substance zéolitique cristalline dénommée ZSM—48 ayant un réseau consistant en tétrahèdres $SiO_4$ réticulés par la mise en commun d'atomes d'oxygène, ayant la formule de composition suivante, exprimée en moles d'oxydes par 100 moles de silice:

$$(0 \text{ à } 15)(RN)_2O\text{: } (0 \text{ à } 15)M_{2/n}O\text{: } (0 \text{ à } 2)Al_2O_3\text{: } (100)SiO_2$$

dans laquelle M est au moins un cation de valence n et RN est un composé organique en $C_1$—$C_{20}$ ayant au moins un groupe fonctionnel amine de pKa $\geqslant 7$ et caractérisée par un diagramme de diffraction de rayons X ayant au moins les distances interplanaires et les intensités relatives suivantes:

| d(A) | intensité relative |
|---|---|
| 11,8 $\pm$ 0,2 | faible — très forte |
| 10,2 $\pm$ 0,2 | faible — moyenne |
| 7,2 $\pm$ 0,15 | faible |
| 4,2 $\pm$ 0,08 | très forte |
| 3,9 $\pm$ 0,08 | très forte |
| 3,6 $\pm$ 0,06 | faible |
| 3,1 $\pm$ 0,05 | faible |
| 2,85 $\pm$ 0,05 | faible |

2. Un procédé selon la revendication 1, dans lequel le toluène est dismuté à une température de 350 à 750°C sous une pression de 10,325 à 10132,5 kPa et a une vitesse spatiale horaire en poids de 0,25 à 20.

3. Un procédé selon la revendication 1, dans lequel on met en contact le toluène et un agent méthylant avec le catalyseur à une température de 250 à 750°C sous une pression de 10,1325 à 10132,5 kPa et à une vitesse spatiale horaire en poids de 0,1 à 2000, le rapport molaire de l'agent méthylant au toluène étant de 0,05 à 5.

4. Un procédé selon la revendication 3, dans lequel l'agent méthylant est le méthanol et la mise en contact est effectuée à 325—600°C, avec une vitesse spatiale horaire en poids de 1 à 100 et un rapport molaire méthanol/toluène de 0,1 à 2.

5. Un procédé selon la revendication 1, dans lequel on met en contact le toluène et un agent éthylant avec le catalyseur à une température de 200—600°C sous une pression de 10,1325 à 10132,5 kPa, avec une vitesse spatiale horaire en poids de 1 à 100 et un rapport molaire agent éthylant/toluène de 0,5 à 5.

6. Un procédé selon la revendication 5, dans lequel l'agent éthylant est l'éthylène et la mise en contact est effectuée à 300—500°C sous une pression de 10,1325 à 5066,25 kPa et avec un rapport molaire éthylène/toluène de 0,1 à 2.

7. Un procédé selon la revendication 1, dans lequel on met en contact le toluène et un agent propylant avec le catalyseur à une température de 150 à 500°C sous une pression de 10,1325 à 10132,5 kPa, avec une vitesse spatiale horaire en poids de 1 à 100 et un rapport molaire agent propylant/toluène de 0,5 à 5.

8. Un procédé selon la revendication 7, dans lequel l'agent propylant est le propylène et la mise en contact est effectuée à 200—400°C sous 10,1325 à 5066,25 kPa et avec un rapport molaire propylène/toluène de 0,1 à 2.

9. Un procédé selon la revendication 3, dans lequel l'agent méthylant est le chlorure ou le bromure de méthyle, l'éther diméthylique, le carbonate de méthyle, le sulfure de diméthyle ou des oléfines légères.

10. Un procédé selon la revendication 5, dans lequel l'agent éthylant est l'éthanol, l'éther éthylique ou le chlorure ou le bromure d'éthyle.

11. Un procédé selon la revendication 7, dans lequel l'agent propylant est l'alcool isopropylique, l'éther isopropylique ou le chlorure ou le bromure d'isopropyle.

12. Un procédé selon l'une quelconque des revendications précédentes, dans lequel la zéolite ZSM—48 a un rapport molaire silice/alumine plus grand que 30.

13. Un procédé selon l'une quelconque des revendications précédentes, dans lequel la zéolite ZSM—48 a un rapport silice/alumine plus grand que 50.

14. Un procédé selon l'une quelconque des revendications précédentes, dans lequel la zéolite ZSM—48 a un rapport silice/alumine plus grand que 400.

15. Un procédé selon l'une quelconque des revendications précédentes, dans lequel la zéolite ZSM—48 est sous la forme hydrogène.